# EUROPEAN PATENT SPECIFICATION

(11) **EP 1 366 664 B1**
(45) Date of publication and mention of the grant of the patent: **17.09.2008**
(21) Application number: 01949983.9
(22) Date of filing: 16.07.2001
(51) Int. Cl.: A01N 59/06, A01N 37/44, A01N 25/00, A23L 3/3526

(54) **ANTIBACTERIAL COMPOSITION**
ANTIBAKTERIELLE ZUSAMMENSETZUNG
COMPOSITION ANTIBACTERIENNE

(30) Priority: 16.02.2001 JP 2001040217
(43) Date of publication of application: 03.12.2003
(73) Proprietor: MURAKASHI LIME INDUSTRY CO., LTD, Aso-gun Tochigi 327-0509 (JP)
(72) Inventor: URANO, Teruo, Sano-City Tochigi 327-0103 (JP); KOBAYASHI, Kazuya, Tochigi-City Tochigi 328-0031 (JP); INA, Sachio, Aso-Gun Tochigi 327-0312 (JP)
(74) Representative: Hoarton, Lloyd Douglas Charles
(86) International application number: PCT/JP2001/006123
(87) International publication number: WO 2002/063963

(56) References cited:
- WO-A-01/32037
- WO-A-02/30948
- WO-A1-01/58267
- JP-A- 4 316 506
- JP-A- 8 154 640
- JP-A- 8 256 703
- JP-A- 8 322 529
- JP-A- 58 138 368
- JP-A- 2000 159 591
- JP-A- 2000 189 129
- JP-A- 2001 192 310
- BUTA J.G. ET AL.: 'Extending storage life of fresh-cut apples using natural products and their derivatives' JOURNAL OF AGRICULTURAL AND FOOD CHEMISTRY vol. 47, no. 1, 1999, pages 1 - 6, XP001055093

## Description

### Field of the Invention

The present invention relates to antibacterial compositions exhibiting low biological and environmental loads and high water-solubility.

### Background Art

Antibacterial agents against fungi, mold, yeast, or the like are widely utilized in the fields of construction materials, lumber, leather, paints, plastics, film, ceramics, paper, pulp, foods, medical supplies, cosmetics, or the like. The antibacterial agents are roughly divided into those of organic type and inorganic type.

An organic type antibacterial agent includes such types as phenolic, halogen, aldehyde, ester, carboxylic acid, nitryl and organic metal, and is used as disinfectants, antiseptics, and fungicides, exhibiting a remarkable bactericidal effect for fungi. However, they have heavy biological and environmental loads, and not a few of them are skin-stimulative, mucosa-stimulative, eye-stimulative and toxic. Moreover, the preservatives used as food additives include carboxylic acid such as sorbic acid, propionic acid, and sal of them, but their use is strictly restricted despite of their excellent effects.

The inorganic antibacterial agent utilizes silver, copper, or titanium oxide and they are used by being fixed in a porous material such as phosphate type salt ceramics or zeolite, or by being kneaded in plastic, textile, or the like. Although the feature of inorganic antibacterial agents has wide antibacterial spectrum, their drawbacks are that the materials are expensive and contain harmful substances, and that in the case of titanium oxide, it needs to be irradiated with light. The other antibacterial agents, ceramic type antibacterial agents such as calcium oxide, magnesium oxide, zinc oxide, or the like are known.

The above-mentioned antibacterial agents are used for the usage according to the characteristic of each material. Especially, among these antibacterial materials, the antibacterial activities of calcium oxide, magnesium oxide, zinc oxide or the like, which have high safety to living bodies, also contain mineral elements essential to the living bodies and are capable of being taken orally, have considerable attention.

On the other hand, amino acid, for example, glycine exhibits a growth-inhibitory function to various microorganisms, and is used as a quality improving agent by being added to foods for suppressing multiplication of putrefactive bacteria or the like, however, it cannot be said that the effect is sufficient.

The aforementioned ceramic type antibacterial agents exhibit a low biological and environmental load and can also be taken orally. However, these are slightly water-soluble solid matters, for example, calcium oxide and calcium hydroxide have small amounts of water-solubility such as 1400 µg/gH₂O and 1800g/gH₂O, (at 20°C in both cases) respectively, and only dilute solutions can be obtained, therefore, they cannot exert a sufficient antibacterial effect. Moreover, even though a bacterial population is decreased in general, bacteria multiply further in a few hours. From this point of view, a larger quantity of additive agent than the minimum inhibitory concentration (MIC) is required to expect a lasting antibacterial effect. Therefore, in case of adopting a calcium oxide or a calcium hydroxide, they get to a saturated concentration or more dense and cannot be recognized as water solutions, and it brings about poor effectiveness as preservatives for foods.

Therefore, they are used as suspension liquids, and excessive calcium oxide and calcium hydroxide become slurry to be precipitated out on the bottom of the container. Moreover, in a dipping process, since a solid matter remains in the object or the pH of strong alkali stays at 12.5, objects for the use are limited, at the same time they easily react with carbon dioxide in the air to produce calcium carbonate, causing a problem that the antibacterial effect does not continue.

Moreover, when powder of calcium oxide or calcium hydroxide is mixed in a food as a preservative, the mixture is strongly alkaline to be a problem in the taste, and a chemical change is triggered with the object, so the usages are further limited.

Besides, since MIC is a minimum inhibitory concentration of fungi, it is an applicable figure for bacteriostasis in foods. In a case of colon bacilli, for example, a food poisoning breaks out at a bacterial population of 10⁶ - 10¹⁰ pcs/g.

The purpose of the present invention is to provide antibacterial compositions which contain calcium oxide, calcium hydroxide or a mixture of them in high concentrations, and of which the water solution exhibits a low pH.

JP 2000-189129 discloses a preserving agent for food comprising an alkaline metal carbonate.

JP 08-322529 discloses a further food preservative containing baked oyster shell, glycine and a soft roe protein.

JP 08-258703 discloses a quality improver for food comprising calcium oxide to create alkaline pH.

JP 08-154640 discloses an antimicrobial agent for food comprises acidic acid, glycine and baked calcium.

According to the present invention, there is provided the use of a composition either or both of a calcium oxide/alpha-amino acid chelate compound or a calcium hydroxide/alpha-amino acid chelate compound, wherein a molar ratio of calcium metal to amino acid ligand in the chelate is 1:2, as an antibacterial agent.

Preferably, either the calcium oxide/alpha-amino acid chelate compound is produced by reacting calcium oxide or calcium hydroxide or a mixture of both with alpha-amino acid under the presence of water.

Advantageously the alpha-amino acid is selected from one or more of glycine, alanine, valine, leucine, isoleucine, threonine, cystine, asparagines, glutamine, lysine, and histidine.

### Disclosure of the Invention

The antibacterial compositions relating to the present invention are those which contain either or both of a calcium oxide α-amino acid chelate compound or a calcium hydroxide · α-amino acid chelate compound. This antibacterial composition can be obtained by reacting the calcium oxide, the calcium hydroxide, or a mixture of them with the α-amino acid under the presence of water.

Investigating in details a mole ratio of a reaction between the calcium oxide or the calcium hydroxide and the α-amino acid under the presence of water, it turned out to be clear that the calcium oxide or the calcium hydroxide of 1 mole reacts with the α-amino acid of 2 moles. Since these reactants exhibit that the pH is saturated at about 10 irrespective of the dissolving amounts of the calcium oxide or the calcium hydroxide and the electric conductivity of the water solution remains almost unchanged, it is considered to be clear that the calcium oxide or the calcium hydroxide and the α-amino acid produce a water-soluble chelate compound. In this case, it is presumed that each amino acid of two molecules takes part as a bidentate ligand in forming the chelate compound mainly using the calcium oxide or the calcium hydroxide.

The water solution of this calcium oxide α-amino acid chelate compound is able to lower the reactivity against food materials in the range of around pH 10, and it makes possible to provide a new antibacterial composition with high biological safety by solving the problem of the strong alkali of calcium oxide and calcium hydroxide as preservatives.

The amino acid is a generic name of compounds having an amino group (-NH₂) and a carboxyl group (-COOH) in a living body, and a compound in which the amino group and carboxyl group are coupled to the same carbon atom is called an α-amino acid, and the general formula is expressed as; RCH(NH₂)COOH. R is a side chain, and each amino acid has a unique functional group. In general, α-amino acid is simply called amino acid.

The α-amino acid dissolving the calcium oxide, the calcium hydroxide, or a mixture of both in high concentration includes glycine, alanine, valine, leucine, isoleucine, threonine, cysteine, methionine, asparagine, glutamine, lysine, and histidine. Their calcium oxide - amino acid chelate compounds exhibited an antibacterial effect. The other amino acids, namely, serine, phenylalanine, tyrosine, cystine, proline, tryptophan, and arginine reacted neither with the calcium oxide nor with the calcium hydroxide, consequently their high concentration solutions could not be obtained.

Moreover, also in the cases of asparagine acid and glutamine acid both of which are acidic amino acid, the antibacterial effect could not be observed. It was confirmed that these acidic amino acids caused neutralization reaction between the calcium oxide and the acid-base generating aspartate calcium and calcium glutamate as the reactants and that these calcium salts did not have antibacterial property.

Although the calcium oxide or the calcium hydroxide of ordinary marketed commodity may be used as a row material, the smaller the particle size is, the better the reaction efficiency. On the other hand, the α-amino acid may be a mixture for food use or industrial use. Moreover, it does not matter whether the α-amino acid is in a form of a slid matter or a water solution. Although the water to be used may be ion-exchange water, distilled water, and tap water, low hardness water is preferable.

A method for producing the antibacterial composition by reacting the calcium oxide or the calcium hydroxide or a mixture of them with the α-amino acid under the presence of water will be described in the following. Namely, the calcium oxide or the calcium hydroxide or a mixture of them is put to react with the α-amino acid under the presence of water so as to be 1 : 2 a mole ratio of any of them to the α-amino acid. The reaction may be performed at ordinary temperatures. There is no given order for the calcium oxide or the calcium hydroxide and the α-amino acid to be put into water. Namely, any of them may be added into water first, but both should be dissolved completely in the water. Although stirring is not always necessary in this process, the reaction is surely advanced by the stirring. The concentration of the solution is determined by adjusting the amount of adding water. When insoluble portions are observed, they are removed in a conventional method. For example, filter cloth, filter paper, shifter, or the like and also vacuum filtration, pressure filtration, filter filtration, centrifugal filtration, or the like can be listed up for them, and any of these may be applied, if necessary at the time of production, according to the facilities, efficiency and so on.

The antibacterial composition of the present invention is of a water-solution form in which a high concentration of the calcium oxide, the calcium hydroxide, or a mixture of both is made soluble, and it is preferable to adjust the solution to as high concentration as possible economically. It is used by diluting to an appropriate concentration with water as necessary.

When the use of water is inappropriate, the water content of the solution is vaporized by using drying apparatus such as a spray drier and a freeze-drying process so that the solution may be used as water-soluble dried powder. However, since this solution cannot be stable under the condition of temperature at 110°C or higher, the drying process is preferably conducted at temperature of 110°C or lower.

The calcium oxide dissolves in water only 0.14wt% (20°C), and pH is 12.5. However, when the calcium oxide or the calcium hydroxide is made to react with the amino acid in water, it dissolves in of high concentration, for example, in a solution of calcium oxide-glycine type, a high concentration solution dissolving the calcium oxide of 18wt% therein can be obtained, and the pH is about 10.

Even if a -amino acids are low water-soluble amino acids such as leucine and isoleucine, the presence of the calcium oxide makes both materials react with each other to be soluble without leaving any problem. However, each solubility is different according to the kinds of the α-amino acids to be used.

The inventors of the present invention have completed this invention by confirming that a water-soluble calcium oxide - amino acid chelate compound obtained by making the calcium oxide, the calcium hydroxide, or a mixture of them react with the amino acid under the presence of water exhibits an antibacterial action to fungi and can be used as an antibacterial agent.

The antibacterial composition of the present invention thus obtained can be used as the antibacterial agent for construction materials by applying or kneading it to the inside and outside walls, floors, ceilings, sliding doors, or the like of architecture. Moreover, the antibacterial agent can be used as a bacteria remover, a bacterostatic agent, and a cleaning agent for medical equipment, cooking equipment, kitchen sinks, ventilating fans, air-conditioning equipment, floors and inside walls of large scale facilities such as a plant requiring especially micro-organism control, footwear, hand, and fingers.

In addition, the composition can widely be used as the antibacterial agent for basic cosmetics such as milky lotion, massage masks, and cream, hair-dressings, combs, toothbrushes, soles, chamber pots, toilets for pets, sand pits, washing drums, touch panel on a ticket vending machine or the like, receivers, PC keyboards, mice, mats used in gymnasiums, tatami-mats, water-soluble paints, etc.

The antibacterial composition of the present invention can be used as a preservative by being added to foods. Moreover, it does not have an influence on original quality and taste of foods, because its pH is about 10, and practically shows a sufficient effect by being added a low amount. To be more concrete, it can be used by being added to or mixed into the wide range of foods such as bean curd, raw noodles, pickled vegetables, side dishes, boiled rice, ham, sausage, boiled fish, and dried fish. Especially, lactic acid fermentation can be controlled by using the antibacterial agent of the present invention, and by adding the composition to pickled vegetables or the like, a preservative effect is expected and their excessive fermentation can be prevented.

Since the composition of the present invention is produced from the calcium oxide or the calcium hydroxide and the amino acid as the raw materials, the safety is high not only to the environment but also to a human body. Adding to it, since the composition contains a high concentration of the calcium oxide or the calcium hydroxide, it contributes to an enrichment of calcium nutrition in addition to the preservative effect by being mixed into foods.

Moreover, the composition can be used as a cleaning agent for cut vegetables, vegetables after harvest, fruits, eggs, seeds, flowers, or the like. Since the composition of the present invention contains the calcium oxide, the calcium hydroxide, or a mixture of them in high concentration, the processing time can be shorten by adjusting the concentration through diluting with water as necessary. The composition can be used properly as a bacteriostatic agent in a concentration of 0.1wt% or lower, and as a fungi remover in a concentration of 1.0wt% or higher respectively.

### Best Mode for Carrying out the Invention

In the following, the present invention will be described in details based on the embodiments, however, the present invention is not to be restricted to these embodiments. In the examples below, "part(s)" and "%" mean "part(s) by weight" and "% by weight", respectively. The calcium oxide was used after having baked a special grade reagent calcium oxide in an electric oven at 1000°C for 2 hours and confirmed it to be surely calcium oxide by a powder X-ray diffractometry method. As for a calcium hydroxide and an amino acid, special grade reagent chemicals were used. As for water, distilled water was used.

### Embodiment 1

142.53 parts of powdered glycine were added to 100 parts of water with stirring, and then 53.24 parts of powdered calcium oxide were added thereto. As the reaction advanced, the powdered calcium oxide dissolved in the solution of glycine, increasing the transparency of the solution, and the insoluble portion disappeared. After two-hour reaction, a water-soluble calcium oxide - glycine type antibacterial composition was obtained. The calcium oxide concentration of this composition was 18.00% and its pH was 10.8.

### Embodiment 2

9.13 parts of powdered glycine were added to 100 parts of water with stirring, and then 4.50 parts of powdered calcium hydroxide were added thereto. They were put at room temperature for two hours to react with each other, but an insoluble portion found was removed by filtration, and a water soluble antibacterial composition of calcium hydroxide - glycine type was obtained. A calcium hydroxide concentration of this composition was 2.76, and its pH was 10.3.

### Embodiment 3

20.08 parts of powdered alanine were added to 100 parts of water with stirring, and then 6.32 parts of powdered calcium oxide were added thereto. As the reaction advanced, the powdered calcium oxide dissolved in the solution of alanine, the transparency being increased, and an insoluble portion disappeared. After two-hour reaction, a water-soluble antibacterial composition of calcium oxide - alanine type was obtained. The calcium oxide concentration of this composition was 5.00%, and its pH was 10.8.

### Embodiment 4

11.02 parts of powdered alanine were added to 100 parts of water with stirring, and then 4.58 parts of powdered calcium hydroxide were added thereto. They were put at room temperature for two hours to react with each other, but an insoluble portion found was removed by filtration, and a water-soluble antibacterial composition of calcium oxide - alanine type was obtained. The calcium oxide concentration of this composition was 2.75%, and its pH was 10.8.

### Embodiment 5

20.08 parts of powdered valine were added to 100 parts of water with stirring, and then 5.04 parts of powdered calcium oxide were added thereto. As the reaction advanced, the powdered calcium oxide dissolved in the solution of valine, the transparency being increased, and an insoluble portion disappeared. After two-hour reaction, a water-soluble antibacterial composition of calcium oxide - valine type was obtained. The calcium oxide concentration of this composition was 4.00%, and its pH was 11.4.

### Embodiment 6

4.96 parts of powdered leucine were added to 100 parts of water with stirring, and then 1.06 parts of powdered calcium oxide were added thereto. As the reaction advanced, the powdered calcium oxide dissolved in the solution of leucine, the transparency being increased, and an insoluble portion disappeared. After two-hour reaction, a water-soluble antibacterial composition of calcium oxide - leucine type was obtained. The calcium oxide concentration of this composition was 1.00%, and its pH was 11.4.

### Embodiment 7

4.96 parts of powdered isoleucine were added to 100 parts of water with stirring, and then 1.06 parts of powdered calcium oxide were added thereto. As the reaction advanced, the powdered calcium oxide dissolved in the solution of isoleucine, the transparency being increased, and an insoluble portion disappeared. After two-hour reaction, a water-soluble antibacterial composition of calcium oxide - isoleucine type was obtained. The calcium oxide concentration of this composition was 1.00%, and its pH was 10.6.

### Embodiment 8

47.00 parts of powdered threonine were added to 100 parts of water with stirring, and then 11.07 parts of powdered calcium oxide were added thereto. As the reaction advanced, the powdered calcium oxide dissolved in the solution of threonine, the transparency being increased, and an insoluble portion disappeared. After two-hour reaction, a water-soluble antibacterial composition of calcium oxide - threonine type was obtained. The calcium oxide concentration of this composition was 7.00%, and its pH was 10.5.

### Embodiment 9

29.44 parts of powdered cysteine were added to 100 parts of water with stirring, and then 6.81 parts of powdered calcium oxide were added thereto. As the reaction advanced, the powdered calcium oxide dissolved in the solution of cysteine, the transparency being increased, and an insoluble portion disappeared. After two-hour reaction, a water-soluble antibacterial composition of calcium oxide - cysteine type was obtained. The calcium oxide concentration of this composition was 5.00%, and its pH was 8.6.

### Embodiment 10

15.80 parts of powdered methionine were added to 100 parts of water with stirring, and then 2.97 parts of powdered calcium oxide were added thereto. As the reaction advanced, the powdered calcium oxide dissolved in the solution of methionine, the transparency being increased, and an insoluble portion disappeared. After two-hour reaction, a water-soluble antibacterial composition of calcium oxide - methionine type was obtained. The calcium oxide concentration of this composition was 2.50%, and its pH was 10.5.

### Embodiment 11

54.68 parts of powdered asparagine were added to 100 parts of water with stirring, and then 11.66 parts of powdered calcium oxide were added thereto. As the reaction advanced, the powdered calcium oxide dissolved in the solution of asparagine, the transparency being increased, and an insoluble portion disappeared. After two-hour reaction, a water-soluble antibacterial composition of calcium oxide - asparagine type was obtained. The calcium oxide concentration of this composition was 7.01%, and its pH was 10.5.

### Embodiment 12

64.57 parts of powdered glutamine were added to 100 parts of water with stirring, and then 12.39 parts of powdered calcium oxide were added thereto. As the reaction advanced, the powdered calcium oxide dissolved in the solution of asparagine, the transparency being increased, and an insoluble portion disappeared. After two-hour reaction, a water-soluble antibacterial composition of calcium oxide - glutamine type was obtained. The calcium oxide concentration of this composition was 7.00%, and its pH was 11.0.

### Embodiment 13

5.56 parts of powdered lysine were added to 100 parts of water with stirring, and then 1.07 parts of powdered calcium oxide were added thereto. As the reaction advanced, the powdered calcium oxide dissolved in the solution of lysine, the transparency being increased, and an insoluble portion disappeared. After two-hour reaction, a water-soluble antibacterial composition of calcium oxide - lysine type was obtained. The calcium oxide concentration of this composition was 1.00%, and its pH was 10.3.

### Embodiment 14

12.73 parts of powdered histidine were added to 100 parts of water with stirring, and then 2.30 parts of powdered calcium oxide were added thereto. As the reaction advanced, the powdered calcium oxide dissolved in the solution of hystidine, the transparency being increased, and an insoluble portion disappeared. After two-hour reaction, a water-soluble antibacterial composition of calcium oxide - histidine type was obtained. The calcium oxide concentration of this composition was 2.00%, and its pH was 10.9.

### Embodiment 15

8.33 parts of powdered glycine and 9.88 parts of powdered alanine were added to 100 parts of water with stirring, and then 6.22 parts of powdered calcium oxide were added thereto. As the reaction advanced, the powdered calcium oxide dissolved in the solution of amino acid, the transparency being increased, and an insoluble portion disappeared. After two-hour reaction, a water-soluble antibacterial composition of calcium oxide - mixed amino acid type was obtained. The calcium oxide concentration of this composition was 5.00%, and its pH was 10.3.

### Embodiment 16

12.83 parts of powdered glycine and 8.50 parts of powdered methionine were added to 100 parts of water with stirring, and then 6.39 parts of powdered calcium oxide were added thereto. As the reaction advanced, the powdered calcium oxide dissolved in the solution of an amino acid, the transparency being increased, and an insoluble portion disappeared. After two-hour reaction, a water-soluble antibacterial composition of calcium oxide - mixed amino acid type was obtained. The calcium oxide concentration of this composition was 5.00%, and its pH was 10.4.

### Embodiment 17

The individual antibacterial activities of the antibacterial compositions produced in the embodiments 1 through 16, and those of a calcium oxide, a calcium hydroxide, and the α-amino acids used as the raw materials were measured as the minimum inhibitory concentration (MIC) against colon bacilli; Escherichia coli IFO 3301 (Gram-negative bacteria) and yellow Staphylococci; Staphylococcus aureus IFO 12732 (Gram-positive bacteria).

A flat plate for measuring susceptibility was prepared by adding and mixing the a calcium oxide and a calcium hydroxide used as the antibacterial compositions and raw materials for the embodiments 1 through 16 to a sterilized and dissolved agar medium for measuring susceptibility (a culture medium for susceptibility disk N made by Nissui Pharmaceutical Co., Ltd.) so that the compositions are equivalent to 500 to 5,000 µg/ml calcium oxide. Additionally, as to the other α-amino acids used as the raw materials, flat plates for measuring susceptibility each of which contains a 500 to 20,000µ g/ml α-amino acid were prepared. The provided test bacteria were inoculated into an enrichment medium (Bouillon for measuring MIC susceptibility made by Nissui Pharmaceutical Co., Ltd.), and after they were incubated at 35°C ± 1°C for 18 to 20 hours (the number of the bacteria multiplies up to 10⁸ to 10⁹/ml), it was diluted on the enrichment medium so that the number of bacteria was adjusted to 10⁶/ml, and was adopted as a bacterial liquid for inoculation. The bacterial liquid for inoculation was drawn in lines and painted 1 to 2cm on each flat plate for measuring susceptibility with a plastic loop, and incubated at 35°C ± 1°C for 18 to 20 hours. The lowest concentrations in which the painted parts were blocked to grow were defined as the minimum bacteriostasis concentrations of the antibacterial compositions, and the calcium oxide, the calcium hydroxide, and the α-amino acids used as the raw materials in the embodiments 1 through 16. The results are shown in Table 1 and Table 2.

**Table 1**

| | Minimum Inhibitory Concentration CaO; µg/ml | |
|---|---|---|
| Name of Strain Composition | Escherichia coli IFO 3301 (Gram-negative bacteria) | Staphylococcus aureus IFO 12732 (Gram-positive bacteria) |
| Embodiment 1 | 750 | 700 |
| Embodiment 2 | 1250 | 1000 |
| Embodiment 3 | 1000 | 1000 |
| Embodiment 4 | 1250 | 1000 |
| Embodiment 5 | 1250 | 1000 |
| Embodiment 6 | 1250 | 1000 |
| Embodiment 7 | 1250 | 1250 |
| Embodiment 8 | 1500 | 1500 |
| Embodiment 9 | 2500 | 5000 |
| Embodiment 10 | 800 | 1250 |
| Embodiment 11 | 2500 | 2500 |
| Embodiment 12 | 2500 | 2500 |
| Embodiment 13 | 1250 | 1250 |
| Embodiment 14 | 2500 | 2500 |
| Embodiment 15 | 1250 | 1000 |
| Embodiment 16 | 1250 | 1000 |

**Table 2**

| | Minimum Inhibitory Concentration CaO; µg/ml | |
|---|---|---|
| Name of Strain Composition | Escherichia coli IFO 3301 (Gram-negative bacteria) | Staphylococcus aureus IFO 12732 (Gram-positive bacteria) |
| Comparison 1 CaO | 1250 | 1000 |
| Comparison 2 Ca(OH)₂ | 900 | 850 |
| Comparison 3 glycine | > 10000 | > 10000 |
| Comparison 4 alanine | > 10000 | > 10000 |
| Comparison 5; valine | > 10000 | > 10000 |
| Comparison 6 leucine | > 10000 | > 10000 |
| Comparison 7 isoleucine | > 10000 | > 10000 |
| Comparison 8 threonine | > 20000 | > 20000 |
| Comparison 9 cystine | > 20000 | 20000 |
| Comparison 10 Methionine | > 10000 | > 10000 |
| Comparison 11 asparagine | > 20000 | > 20000 |
| Comparison 12 glutamine | > 20000 | > 20000 |
| Comparison 13 lysine | 20000 | > 20000 |
| Comparison 14; hystidine | > 20000 | > 20000 |

From the results shown in Table 1 and Table 2, it is found out that the compositions in accordance with the present invention are excellent in antibacterial effect.

### Embodiment 18

Further, the antibacterial spectra of the embodiment 1, embodiment 3, embodiment 10, and comparison 1 were examined with the minimum bacteriostasis concentrations against Enterococcus faecalis IFO 12964, Bacillus cereus IFO 13494, Lactobacillus plantarum (lactic acid bacterium) IFO 3070, and Clostridium perfringens NCTC 8283 as Gram-positive bacteria, and Salmonella entriditis IFO 3313, and Pseudomonas aeruginosa IFO 13275 as Gram-negative bacteria, and Saccharomyces cerevisiae IFO 1950. The minimum inhibitory concentrations to these typical kinds of bacteria were measured, however, the antibacterial compositions of the present invention are not to be restricted to the kinds of bacteria shown in the embodiments. Although the measuring method was similar to that of the embodiment 17, as the culture media for measuring susceptibility, Muller Hinton Agar (Difco) was used for enterococcus, cereus, salmonella, and pseudomonas aeruginosa; Brain Heart Infusion Agar (Difco) was used for lactobacillus; GAM agar medium (Nissui Pharmaceutical Co., Ltd.) was used for welsh; and Sabouraud Glucoss agar medium (Eiken Chemical Co., Ltd.) was used for saccharomyces. The test results are shown in Table 3.

**Table 3**

| | Minimum Inhibitory Concentration CaO (µg/ml) | | | |
|---|---|---|---|---|
| Compositi on Name of Strain | Embodim ent 1 | Embodim ent 3 | Embodim ent 10 | Comparis on 1 (reference value) |
| Salmonell a enteritidis | 2500 | 2500 | 1250 | 2500 |
| Pseudom onas aeruginosa | 2500 | 2500 | 2500 | 2500 |
| Enterococ cus faecalis | 2500 | 5000 | 5000 | 5000 |
| Bacillus cereus | 2500 | 2500 | 2500 | 2500 |
| Lactobac hillus faecalis | 313 | 313 | 625 | 313 |
| Clostridiu m perfringens | 2500 | 5000 | 2500 | 5000 |
| Saccgaro myces cervisiae | 1250 | 625 | 1250 | 625 |

From the above results, it has been found out that the compositions of the present invention can be used as antibacterial agents according to the antibacterial actions to these bacteria. The values of the comparison 1 in Table 3 are not less than the saturated solubility of calcium oxide (1400 µg CaO /gH₂O, 20°C: 0.14%), and as to the case that values are not less than 1400 µg, the minimum inhibitory concentrations were measured by developing the antibacterial compositions in the state of suspension on the culture media. For this reason, it is impossible to simply compare the comparison 1 with the embodiments 1, 3, 10, therefore, the results are mentioned as reference values.

### Embodiment 19

A dipping sterilization process was performed on fresh vegetables by using the antibacterial composition of a calcium oxide - amino acid type of the embodiment 1 which was adjusted equivalent to the concentration 1% of calcium oxide by diluted 18 times with water. The amount of bean sprouts for dipping was made to 1/100 of the processing liquid by weight, and the number of general bacteria and Escherichia coli group on the bean sprouts before dipping, 30 minutes after dipping and 60 minutes after dipping were checked. For counting the number of bacteria, 10g bean sprouts were aseptically collected on a stomacher after dipping for a prescribed time, and then an undiluted test solution was made by adding 90g sterilized phosphate buffer physiological saline thereto and stomaching it. The undiluted test solution was diluted stepwise by 10-times steps, and 1ml of the solution is collected at each step to be separately infused into laboratory dishes, and a standard agar medium (Nissui Pharmaceutical Co., Ltd.) for the general bacteria, and an XMG agar medium (Nissui Pharmaceutical Co., Ltd.) for the Escherichia coli group were poured into the laboratory dishes respectively to mix and dilute therewith. After the solidification of the culture media, they were inverted to be cultured at 35°C, and the colonies, which appeared after 48 hours as to the standard agar media and also after 20 hours as to the XMG agar media, were counted and multiplied by the dilution factor, and the result were adopted as the general number of bacteria, and the number of bacteria of the Escherichia coli group of the bean sprouts, respectively. Moreover, as examples for comparison, similar dipping process of bean sprouts was performed also with 1% calcium oxide suspension (comparison 15) and distilled water (comparison 16). The test results are shown in Table 4.

**Table 4**

| Dipping time(min) Treating liquid | Before dipping | 30 | 60 |
|---|---|---|---|
| | General number of bacteria (cfu/g) | | |
| Embodiment 1 | 1.0 x 10⁷ | 1.1 x 10⁶ | 9.2 x 10⁴ |
| Comparison 15 | | 3.7 x 10⁶ | 4.1 x 10⁵ |
| Comparison 16 | | 9.0 x 10⁶ | 7.7 x 10⁶ |

| | Escherichia coli group (cfu/g) | | |
|---|---|---|---|
| Embodiment 1 | 31.6 x 10⁶ | 1.2 x 10⁵ | 1.4 x 10⁴ |
| Comparison 15 | | 3.0 x 10⁵ | 4.3 x 10⁵ |
| Comparison 16 | | 2.6 x 10⁶ | 2.6 x 10⁵ |

When bean sprouts were dipped for 60 minutes in the 18-fold water-diluted composition of the embodiment 1, both numbers of the general bacteria and the Escherichia coli group were decreased approximately by 10² respectively, and it indicates the composition is capable of removing bacteria from vegetables.

### Industrial Applicability

The antibacterial compositions containing high concentration of antibacterial calcium oxide, calcium hydroxide, or a mixture of both, and a water solution thereof with low pH can be obtained.

## Claims

1. Use of a composition containing either or both of a calcium oxide/alpha-amino acid chelate compound or a calcium hydroxide/alpha-amino acid chelate compound, wherein a molar ratio of calcium metal to amino acid ligand in the chelate is 1:2, for the preparation of an antibacterial agent.

2. Use of the composition as claimed in Claim 1, wherein either the calcium oxide/alpha-amino acid chelate compound or the calcium hydroxide/alpha-amino acid chelate compound is produced by reacting calcium oxide or calcium hydroxide or a mixture of both with alpha-amino acid under the presence of water.

3. Use of a composition as claimed in Claim 1 or 2, wherein the alpha-amino acid is selected from one or more of glycine, alanine, valine, leucine, isoleucine, threonine, cystine, asparagines, glutamine, lysine, and histidine, as an antibacterial agent.

## Patentansprüche

1. Verwendung einer Zusammensetzung, die eine Kalziumoxid/alpha-Aminosäurechelat-Verbindung und/oder eine Kalziumhydroxid/alpha-Aminosäurechelat-Verbindung enthält, wobei ein Molverhältnis von Kalziummetall zu Aminosäureligand in dem Chelat 1:2 beträgt, zur Zubereitung eines antibakteriellen Agens.

2. Verwendung der Zusammensetzung nach Anspruch 1, wobei entweder die Kalziumoxid/alpha-Aminosäurechelat-Verbindung oder die Kalziumhydroxid/alpha-Aminosäurechelat-Verbindung durch das Reagieren-Lassen von Kalziumoxid oder Kalziumhydroxid oder einer Mischung von beiden mit alpha-Aminosäure bei Vorhandensein von Wasser erzeugt wird.

3. Verwendung einer Zusammensetzung nach Anspruch 1 oder 2, wobei die alpha-Aminosäure aus einem oder mehreren von Glycin, Alanin, Valin, Leucin, Isoleucin, Threonin, Cystin, Asparaginen, Glutamin, Lysin und Histidin ausgewählt wird, als antibakterieller Agens.

4. Zusammensetzung, die eine Kalziumoxid/ α-Aminosäurechelat-Verbindung und/oder eine Kalziumhydroxid/ α-Aminosäurechelat-Verbindung enthält, wobei ein Molverhältnis von Kalziummetall zu Aminosäureligand in dem Chelat 1:2 beträgt, zur Verwendung als antibakterieller Agens.

## Revendications

1. Utilisation d'une composition contenant un composé chélaté d'oxyde de calcium/acide α-aminé et/ou un composé chélaté d'hydroxyde de calcium/acide aminé, dans laquelle un rapport molaire du métal calcium au ligand acide aminé dans le chélate est de 1:2, pour la préparation d'un agent antibactérien.

2. Utilisation de la composition selon la revendication 1, dans laquelle le composé chélaté d'oxyde de calcium/acide α-aminé ou le composé chélaté d'hydroxyde de calcium/acide aminé est produit par la réaction d'oxyde de calcium ou d'hydroxyde de calcium ou d'un mélange des deux avec un acide α-aminé en présence d'eau.

3. Utilisation d'une composition selon la revendication 1 ou 2, dans laquelle l'acide α-aminé est choisi parmi une ou plusieurs des glycine, alanine, valine, leucine, isoleucine, thréonine, cystine, asparagines, glutamine, lysine et histidine, en tant qu'agent antibactérien.

4. Composition contenant un composé chélaté d'oxyde de calcium/acide α-aminé et/ou un composé chélaté d'hydroxyde de calcium/acide aminé, dans lequel un rapport molaire du métal calcium au ligand acide aminé dans le chélate est de 1:2, pour une utilisation en tant qu'agent antibactérien.
